# EUROPÄISCHE PATENTANMELDUNG

(11) **EP 1 882 516 A2**
(43) Veröffentlichungstag der Anmeldung: **30.01.2008**
(21) Anmeldenummer: 07010663.8
(22) Anmeldetag: 30.05.2007
(51) Int. Cl.: B01F 17/34, A61K 8/02, B01F 17/42

(54) **Verfahren zur Herstellung feinteiliger Öl-in-Wasser-Emulsionen**

(30) Priorität: 01.07.2006 DE 102006030532
(71) Anmelder: Evonik Goldschmidt GmbH, 45127 Essen (DE)
(72) Erfinder: Venzmer, Joachim, Dr., 45239 Essen (DE); Meyer, Jürgen, Dr., 48143 Münster (DE); Scheuermann, Ralph, Dr., 45359 Essen (DE)

(57) **Zusammenfassung**

Gegenstand der Erfindung ist ein Verfahren zur Herstellung von feinteiligen Öl-in-Wasser Emulsionen, die Öl, Wasser und mindestens einen Emulgator enthalten, dadurch gekennzeichnet, dass es einen Schritt
A) Herstellen einer Mischung 2, die Ö1, Wasser, mindestens einen Emulgator und mindestens eine kosmotrope Substanz aufweist, durch Mischen von Öl, Wasser, mindestens einem Emulgator und mindestens einer kosmotropen Substanz, wobei die Phaseninversionstemperatur PIT2 dieser Mischung (Winsor-IV-System) kleiner ist als die Phaseninversionstemperatur PIT1 einer Mischung 1 (Winsor-IV-System), die keine kosmotropen Substanzen und ansonsten die gleiche Zusammensetzung wie Mischung 2 aufweist,
und nachfolgend einen Schritt
B) Zugabe eines Verdünnungsmittels zur Mischung 2 zur Überführung dieser Mischung in eine Emulsion 3, wobei die Menge des zugegebenen Verdünnungsmittels so gewählt ist, dass die erhaltene Emulsion 3 bei einer vorgegebenen Temperatur nicht im Winsor-IV-Phasengebiet liegt,
umfasst.

## Beschreibung

Die vorliegende Erfindung beschäftigt sich mit Verfahren zur Herstellung feinteiliger Öl-in-Wasser-Emulsionen. Sie beschäftigt sich insbesondere mit Verfahren, in denen die Phaseninversionstemperatur (PIT) des jeweils vorliegenden Systems durch Zugabe kosmotroper Substanzen beeinflusst wird.

Sowohl für kosmetische, dermatologische und pharmazeutische Formulierungen als auch in wässrigen Formulierungen für die Bereiche Haushalt und Industrie werden in gewissen Anwendungsbereichen vorzugsweise Öl-in-Wasser(O/W)-Emulsionen verwendet.

Die konventionell hergestellten Emulsionen weisen Tröpfchengrößen im µm-Bereich auf und haben dadurch den Nachteil, dass sie ohne Zugabe zusätzlicher Stabilisatoren nicht stabil sind, d.h. sie neigen zur Phasentrennung. Deshalb sind mit herkömmlichen Methoden insbesondere langzeitstabile Emulsionen mit niedriger Viskosität nur sehr vereinzelt herstellbar.

Eine Alternative sind thermodynamisch stabile Mikroemulsionen. Diese sind zwar separationsstabil, existieren aber nur in engen Konzentrations- und Temperaturbereichen, die nicht für alle Anwendungsbereiche ausreichend sind.

Die nach dem Phasen-Inversions-Temperatur-Verfahren (PIT-Verfahren)(K. Shinoda, H. Kunieda; Encyclopedia of Emulsion Technology; Vol.1 (1983), 337-367) hergestellten Emulsionen sind ebenfalls äußerst feinteilig, d.h. sie gewährleisten aufgrund ihrer Tröpfchengrößen im Bereich von ca. 20 bis 200 nm eine hervorragende Stabilität in großen Temperatur- und Konzentrationsbereichen.

Bei diesem Verfahren laufen nach gängigen Modellvorstellungen folgende Vorgänge ab:

Bei Raumtemperatur entsteht aus Öl, Wasser und Emulgatoren eine Zweiphasenmischung, enthaltend eine O/W-Mikroemulsion und eine Ölphase (Winsor-I-Typ, W I).

Zur Erzielung eines Einphasengebietes (Winsor-IV-Typ, W IV) ist die Erhöhung der Emulgatorkonzentration allein nicht ausreichend; eine Erhöhung der Temperatur ist zwingend erforderlich. Bei einer systemabhängigen Mindesttemperatur der Phaseninversionstemperatur (PIT) bildet sich eine bikontinuierliche, homogene Mischphase (Winsor-IV-Typ), bei der eine Umkehrung der Phasen von O/W in W/O stattfindet.

Bei weiterer Temperaturerhöhung geht das homogene Winsor-IV-System in ein zweiphasiges Winsor-II-System (W II) über, in dem eine W/O-Mikroemulsion mit einer überschüssigen Wasserphase im Gleichgewicht steht.

In der Technik wird nunmehr davon Gebrauch gemacht, dass bei einer sehr schnellen Abkühlung sich eine Mikroemulsion vom Typ W IV ausbilden kann, die nach Phaseninversion in O/W dann praktisch "eingefroren" wird, so dass eine weitere Umwandlung in den Typ W I unterbleibt.

Erhalten werden so äußerst stabile feinteilige Emulsionskonzentrate, die unbegrenzt mit Wasser verdünnbar sind.

Dieses Verfahren war bisher die einzige Möglichkeit, derartige Emulsionen auch im technischen Maßstab herzustellen.

Nachteilig ist, dass bei diesem Verfahren die Mischung der Komponenten über die Phaseninversionstemperatur aufgeheizt werden muß, um die bei Raumtemperatur vorliegende O/W-Emulsion in eine W/O-Emulsion umzukehren und durch anschließendes Abkühlen eine feinteilige O/W-Emulsion herzustellen. Der erforderliche Energieaufwand zur Aufheizung und effektiven Abkühlung ist erheblich und unökonomisch.

Es bestand daher ein Bedarf nach einem kostengünstigen Verfahren zur Herstellung feinteiliger Emulsionen, die eine hervorragende Stabilität in großen Temperatur- und Konzentrationsbereichen aufweisen.

Diese Aufgabe wird gelöst durch ein Verfahren, bei dem in einer ersten Stufe die sogenannte Phaseninversionstemperatur (PIT) durch Verwendung von kosmotropen Substanzen mindestens bis auf das Niveau der Raumtemperatur bzw. der Anwendungstemperatur gesenkt und in einer zweiten Stufe durch Zugabe von Verdünnungsmitteln wieder, vorzugsweise auf das ursprüngliche Niveau, erhöht wird.

Die kosmotropen Substanzen ersetzen in diesem Verfahren den Schritt der Temperaturerhöhung.

Durch Zugabe von ausreichenden Mengen an Verdünnungsmitteln, erfindungsgemäß vorzugsweise Wasser oder wässrige, gegebenenfalls alkoholische Lösungen, wird die für die Senkung der Phaseninversionstemperatur erforderliche Mindestkonzentration der kosmotropen Substanzen (KS) unterschritten, so dass das ursprüngliche Temperaturniveau wiederhergestellt wird. Die Verdünnung erfolgt dabei so schnell, dass - ähnlich wie bei der schnellen Temperatursenkung - keine Rückbildung der Emulsion in den Winsor-Typ-W I erfolgt.

Da dieses Verfahren im Wesentlichen also auf einer Deaktivierung der kosmotropen Substanzen beruht ("Quenchen"), kann, in Anlehnung an das temperaturgesteuerte PIT-Verfahren, im vorliegenden Fall von einem PSQ-Verfahren (Phase Shift by Quenching) gesprochen werden.

Ein Gegenstand der Erfindung ist daher ein Verfahren zur Herstellung von feinteiligen Öl-in-Wasser-Emulsionen, die Öl, Wasser und mindestens einen Emulgator enthalten, und die bevorzugt bei Umgebungs-, Verarbeitungs- oder Verwendungstemperatur kinetisch stabil sind, dadurch gekennzeichnet, dass es einen Schritt
A) Herstellen einer Mischung 2, die Öl, Wasser, mindestens einen Emulgator und mindestens eine kosmotrope Substanz aufweist, durch Mischen von Öl, Wasser, mindestens einem Emulgator und mindestens einer kosmotropen Substanz, wobei die Phaseninversionstemperatur PIT2 dieser Mischung (Winsor-IV-System) kleiner ist als die Phaseninversionstemperatur PIT1 einer Mischung 1 (Winsor-IV-System), die keine kosmotropen Substanzen und ansonsten die gleiche Zusammensetzung wie Mischung 2 aufweist,
   und nachfolgend einen Schritt
B) Zugabe eines Verdünnungsmittels zur Mischung 2 zur Überführung dieser Mischung in eine Emulsion 3, wobei die Menge des zugegebenen Verdünnungsmittels so gewählt ist, dass die erhaltene Emulsion 3 bei einer vorgegebenen Temperatur nicht im Winsor-IV-Phasengebiet liegt,
umfasst, sowie auf diese Weise erhältliche feinteilige Öl-in-Wasser-Emulsionen.

Bevorzugt wird in dem erfindungsgemäßen Verfahrens eine feinteilige Öl-in-Wasser-Emulsion 3 hergestellt, die eine mittlere Teilchengröße von kleiner 1 µm, bevorzugt von 10 bis 500 nm, besonders bevorzugt von 15 bis 300 nm und ganz besonders bevorzugt von 60 bis 200 nm, aufweist.

Als Mischung 2 wird in dem erfindungsgemäßen Verfahren vorzugsweise eine Mikroemulsion hergestellt.

In Schritt B) des erfindungsgemäßen Verfahrens werden zu 1 Massenteil der Mischung 2 vorzugsweise mindestens 1, bevorzugt mindestens 5 und besonders bevorzugt mindestens 10, Massenteile Verdünnungsmittel zugegeben.

In dem erfindungsgemäßen Verfahren wird in Schritt B) vorzugsweise soviel Verdünnungsmittel zugegeben, dass die Übergangstemperatur, bei der die Emulsion 3 in das Winsor-IV-Phasengebiet übergeht, zumindest 1 K, vorzugsweise 10 K, besonders bevorzugt 40 K, oberhalb der Umgebungs-, der Verarbeitungs- oder Verwendungstemperatur liegt.

Als Verdünnungsmittel können in Schritt B) z.B. Wasser oder eine wässrige Lösung eingesetzt werden.

Eine Variante des erfindungsgemäßen Verfahrens zur Herstellung der feinteiligen Öl-in-Wasser-Emulsionen besteht darin, dass als Emulsion 3 eine bei Umgebungs-, Verarbeitungs- oder Verwendungstemperatur kinetisch stabile Emulsion hergestellt wird, wobei in Schritt A) eine thermodynamisch stabile und makroskopisch homogene Mischung aus Wasser, Öl, mindestens einem Emulgator und mindestens einer kosmotropen Substanz nach üblichen Verfahren hergestellt wird, und wobei der Schritt A) bei einer Temperatur durchgeführt wird, die kleiner als die Phaseninversionstemperatur PIT1 der Mischung 1 ohne Zugabe der kosmotropen Substanzen ist.

Eine weitere Variante des erfindungsgemäßen Verfahrens zur Herstellung der feinteiligen Öl-in-Wasser-Emulsionen besteht darin, dass als Emulsion 3 eine bei Umgebungs-, Verarbeitungs- oder Verwendungstemperatur kinetisch stabile Emulsion hergestellt wird, wobei Schritt A) das Zumischen mindestens soviel zumindest einer kosmotropen Substanz zu einer Mischung 1, die Öl, Wasser und mindestens einen Emulgator enthält und die eine Phaseninversionstemperatur PIT1 aufweist (Winsor-IV-System), so dass eine Mischung 2 erhalten wird, deren Phaseninversionstemperatur (Winsor-IV-System) PIT2 kleiner ist als PIT1, umfasst.

Eine weitere Variante des erfindungsgemäßen Verfahrens zur Herstellung der feinteiligen Öl-in-Wasser-Emulsionen besteht darin, dass als Emulsion 3 eine bei Umgebungs-, Verarbeitungs- oder Verwendungstemperatur kinetisch stabile Emulsion hergestellt wird, wobei in Schritt A) aus Wasser, Öl, mindestens einem Emulgator und mindestens einer kosmotropen Substanz eine W/O-Mikroemulsionsphase im Gleichgewicht mit überschüssiger Wasserphase (Typ W II) nach üblichen Verfahren ohne Überschreitung der ursprünglichen Phaseninversionstemperatur hergestellt wird.

Wie dargelegt, bewirkt die kosmotrope Substanz die Absenkung der Phaseninversionstemperatur. Das Maß der Absenkung ist abhängig von der Art und der Menge der kosmotropen Substanz. Es wurde gefunden, dass die Absenkung der Phaseninversionstemperatur im Wesentlichen proportional zur eingesetzten Menge ist.

Da im zweiten Schritt des Verfahrens der Einfluß der kosmotropen Substanz durch Verdünnung mit insbesondere Wasser wieder aufgehoben wird, kann in jedem Einzelfall die optimale Menge durch einfache orientierende Versuche bestimmt werden. Optimale Menge bedeutet erfindungsgemäß sowohl eine praxisgerechte Menge zur ausreichenden Absenkung der PIT als auch eine praxisgerechte Mindestmenge an Wasser zur schnellen und gezielten Deaktivierung der kosmotropen Substanzen zur Erhöhung der PIT.

Ein weiterer Gegenstand der Erfindung ist die Verwendung des erfindungsgemäßen Verfahrens zur Herstellung von Emulsionen bei der Herstellung von kosmetischen, dermatologischen, pharmazeutischen oder agrochemischen Zubereitungen, bei der Herstellung von Tränktüchern oder in sprühbaren Zubereitungen für Gesichts- und Körperpflege, Babypflege, Sonnenschutz, Make up-Remover, Antiperspiratien/Deodorantien, bei der Herstellung wässriger Formulierungen für Applikationen in den Bereichen Haushalt, Sport, Freizeit und Industrie, bei der Herstellung von Tränktüchern oder in sprühbaren Zubereitungen zur Reinigung und Pflege von Textilien, Leder, Kunststoffen, metallischen und nichtmetallischen Oberflächen sowie bei der Herstellung von sprühbaren Zubereitungen von agrochemischen Formulierungen, die Öle und gegebenenfalls weitere Aktivstoffe, wie Pestizide, enthalten.

Weitere Gegenstände der Erfindung sind durch die Ansprüche gekennzeichnet.

Die erfindungsgemäß mitverwendeten kosmotropen Substanzen im Sinne der Erfindung sind definitionsgemäß Verbindungen, die gemäß den Hofmeisterschen Reihen Anionen, Kationen, Salze sein können oder organische Verbindungen mit hydrophilen Gruppen, insbesondere Hydroxyl- oder Carboxylgruppen.

Anionen sind beispielsweise SO₄²⁻, PO₄³⁻, Citrat, Tatrat oder Acetat.

Kationen sind beispielsweise Al³⁺, Mg²⁺, Ca²⁺, Ba²⁺, Li⁺, Na⁺ oder K⁺.

Salze sind beispielsweise Natriumcitrat, Na₂SO₄, (NH₄)₂SO₄, NaCl oder NH₄SCN.

Organische Verbindungen sind beispielsweise ein- oder mehrwertige Alkohole wie Butanol, Glycerin, Diglycerin, Triglycerin, Zucker, Zuckeralkohole, Zuckersäuren, Hydroxycarbonsäuren wie Milchsäure, Äpfelsäure, Weinsäure, Zitronensäure oder Ascorbinsäure.

Diese Verbindungen können allein oder in Kombination miteinander und/oder untereinander mitverwendet werden.

Zur Erniedrigung der Phaseninversionstemperatur ausreichender Mengen sind abhängig von Art und Menge der jeweils verwendeten Ölkomponente, der Emulgatorkomponenten und der Art der kosmotropen Substanzen. In der Regel sind Mengen im Bereich von 1 bis 50 Gew.-%, vorteilhafterweise von 20 bis 40 Gew.-%, ausreichend. Die jeweils optimalen Mengen sind durch wenige einfache orientierende Versuche zu ermitteln.

Erfindungsgemäß verwendbare Öle sind grundsätzlich jede in der Kosmetik zur Herstellung reinigender und pflegender wässriger Emulsionen geeignete Verbindung oder deren Mischungen, wie Mono- und Diester von Mono-/Dicarbonsäuren und Mono-/Dialkoholen, beispielsweise der allgemeinen Formel (I), (II) und (III)

R¹-COOR² (I)

R²-OOC-R³-COOR² (II)

R¹-COO-R³-OOC-R¹ (III)

worin
R¹ eine Alkylgruppe mit 8 bis 22 C-Atomen und
R² eine Alkylgruppe mit 3 bis 22 C-Atomen und
R³ Alkylengruppen mit 2 bis 16 C-Atomen bedeuten, mit der Maßgabe, dass die Gesamtzahl der C-Atome in den Verbindungen (I) bis (III) mindestens 11 beträgt.

Diese Verbindungen sind als kosmetische und pharmazeutische Ölkomponenten bekannt. Unter den Mono- und Diestern dieser Art kommt den bei Raumtemperatur (20°C) flüssigen Produkten die größte Bedeutung zu. Als Ölkörper geeignete Monoester (I) sind z.B. die Isopropylester von Fettsäuren mit 12 bis 22 C-Atomen, wie z.B. Isopropylmyristat, Isopropylpalmitat, Isopropylstearat, Isopropyloleat. Andere geeignete Monoester sind z.B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholgemischen und technischen aliphatischen Carbonsäuren erhältlich sind, z.B. Ester aus gesättigten und ungesättigten Fettalkoholen mit 12 bis 22 C-Atomen und gesättigten und ungesättigten Fettsäuren mit 12 bis 22 C-Atomen, wie sie aus tierischen und pflanzlichen Fetten zugänglich sind. Geeignet sind auch natürlich vorkommende Monoester- bzw. Wachsester-Gemische, wie sie z.B. im Jojobaöl oder im Spermöl vorliegen.

Geeignete Dicarbonsäureester (II) sind z.B. Di-n-butyladipat, Di-n-butylsebacat, Di-(2-ethylhexyl)-adipat, Di-(2-hexyldecyl)-succinat und Di-isotridecylacelaat. Geeignete Diolester (III) sind z. B. Ethylenglykoldioleat, Ethylenglykoldiisotridecanoat, Propylenglykoldi(2-ethylhexanoat), Propylenglykoldiisostearat, Propylen-45-glykol-dipelargonat, Butandioldiisostearat und Neopentylglykoldicaprylat.

Als Ölkörper gut geeignet sind ferner Ester von drei- und mehrwertigen Alkoholen, insbesondere pflanzliche Triglyceride, z.B. Olivenöl, Mandelöl, Erdnußöl, Sonnenblumenöl oder auch die Ester des Pentaerythrits mit z.B. Pelargonsäure oder Ölsäure.

Als Fettsäuretriglyceride können natürliche, pflanzliche Öle, z.B. Olivenöl, Sonnenblumenöl, Sojaöl, Erdnußöl, Rapsöl, Mandelöl, Palmöl, aber auch die flüssigen Anteile des Kokosöls oder des Palmkernöls sowie tierische Öle, wie z.B. Klauenöl, die flüssigen Anteile des Rindertalges oder auch synthetische Triglyceride, wie sie durch Veresterung von Glycerin mit Fettsäuren mit 8 bis 22 C-Atomen erhalten werden, z.B. Triglyceride von Caprylsäure-Caprinsäure-Gemischen, Triglyceride aus technischer Ölsäure oder aus Palmitinsäure-Ölsäure-Gemischen, zur Anwendung kommen.

Bevorzugt eignen sich solche Mono- und Diester und Triglyceride als Ölkomponenten für das erfindungsgemäße Verfahren, die bei Normaltemperatur von 20°C flüssig sind. Es können aber auch höherschmelzende Fette und Ester, die den angegebenen Formeln entsprechen, in solchen Mengen mitverwendet werden, dass die Mischung der Ölkomponenten bei Normaltemperatur flüssig bleibt.

Die Ölkomponente kann auch Kohlenwasserstofföle in untergeordneten Mengen bis zu maximal 25 Gew.-% - bezogen auf die Ölkomponente - enthalten. Geeignete Kohlenwasserstoffe sind vor allem Paraffinöle und synthetisch hergestellte Kohlenwasserstoffe, z.B. flüssige Polyolefine oder definierte Kohlenwasserstoffe, z.B. Alkylcyclohexane, wie z.B. das 1.3-Diisooctylcyclohexan.

Bevorzugt sind Ester von linearen C₈-C₁₈-Fettsäuren mit linearen oder verzweigten C₆-C₂₂-Fettalkoholen bzw. Ester von verzweigten C₂-C₁₃-Carbonsäuren mit linearen oder verzweigten C₆₋C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat.

Daneben eignen sich Ester von linearen C₆-C₂₂-Fettsäuren mit verzweigten Alkoholen, insbesondere 2-Ethylhexanol, Ester von C₁₈-C₃₆-Alkylhydroxycarbonsäuren mit linearen oder verzweigten C₆-C₂₂-Fettalkoholen, insbesondere Dioctylmalate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₈-Fettsäuren, flüssige Mono-/Di/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylylcarbonate, Guerbetcarbonate auf Basis von Fettalkoholen mit 6 bis 18, vorzugsweise 8 bis 10 C-Atomen wie beispielsweise Diethylhexylcarbonat (Tegosoft ® DEC, Goldschmidt GmbH), Ester der Benzoesäure mit linearen und/oder verzweigten C₆-C₂₂-Alkoholen, lineare oder verzweigte, symmetrische oder unsymmetrische Dialkylether mit 6 bis 22 Kohlenstoffatomen pro Alkylgruppe, wie z.B. Dicaprylylether, Ringöffnungsprodukte von epoxidierten Fettsäureestern mit Polyolen, und/oder aliphatische bzw. naphthenische Kohlenwasserstoffe, wie z.B. Squalan, Squalen oder Dialkylcyclohexane, Siliconöle wie Cyclomethicone oder Dimethicone, weiterhin propoxylierte Fettalkohole wie PPG-15-Stearylether, PPG-3-Myristylether und PPG-14-Butylether in Betracht.

Grundsätzlich sind als Emulgatoren alle Verbindungen, wie sie im Stand der Technik als Emulgatoren zur Herstellung von kosmetischen O/W- und W/O-Emulsionen verwendet werden, geeignet. Bevorzugt wird dabei mindestens ein Emulgator eingesetzt, ausgesucht aus der Gruppe der ionischen und nichtionischen Emulgatoren.

Ohne Anspruch auf Vollständigkeit seien aus den bekannten Stoffklassen geeigneter Emulgatorkomponenten zusätzlich die folgenden Vertreter benannt:

Als nichtionische Emulgatoren kommen dabei besonders in Frage Oligo-Alkoxylate von lipophile Reste enthaltenden Grundmolekülen. Diese können sich insbesondere von ausgewählten Vertretern aus den nachfolgenden Klassen der lipophile Reste enthaltenden Grundmoleküle ableiten: Fettalkohole, Fettsäuren, Fettamine, Fettamide, Fettsäure- und/oder Fettalkoholester und/oder -ether, Alkanolamide, Alkylphenole und/oder deren Umsetzungsprodukte mit Formaldehyd sowie weitere Umsetzungsprodukte von lipophile Reste enthaltenden Trägermolekülen mit niederen Alkoxiden. Wie angegeben, können die jeweiligen Umsetzungsprodukte auch wenigstens anteilsweise endgruppenverschlossen sein. Beispiele für Partialester und/oder Partialether mehrfunktioneller Alkohole sind insbesondere die entsprechenden Partialester mit Fettsäuren, beispielsweise von der Art der Glycerinmono- und/oder -diester, Glykolmonoester, entsprechende Partialester oligomerisierter mehrfunktioneller Alkohole, Sorbitanpartialester und dergleichen sowie entsprechende Verbindungen mit Ethergruppierungen. Solche Partialester und/oder -ether können insbesondere auch Grundmoleküle für eine (Oligo)-Alkoxylierung sein.

Bei der Alkoxylierung finden vorzugsweise Ethylenoxid, Propylenoxid, Butylenoxid oder Styroloxid Anwendung.

Besonders bevorzugte nichtionische alkoxylierte Emulgatoren sind:

Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; Glycerinmono- und -diester und Sorbitanmono- und -diester von gesättigten und ungesättigten Fettsäuren mit 6 bis 22 Kohlenstoffatomen und deren Ethylenoxidanlagerungsprodukte; Alkylmono- und -oligoglycoside mit 8 bis 22 Kohlenstoffatomen im Alkylrest und deren ethoxylierte Analoga.
Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht;
kammartig oder endständig modifizierte Siliconpolyether, wie sie etwa durch Hydrosilylierungsreaktionen unter bekannten Bedingungen durch Anlagerung alkenfunktionalisierter Polyether mit bevorzugt 2 bis 100 Mol Ethylenoxid und/oder Propylenoxid erhältlich sind. Die endständigen Hydroxylgruppen solcher Polyether können dabei gegebenenfalls auch alkylterminiert sein (insbesondere methylterminiert).

Weiterhin können als nichtionische Emulgatoren auch Verwendung finden:

Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen; Partialester auf Basis linearer, verzweigter, ungesättigter bzw. gesättigter C₆/₂₂-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose); Polysiloxan-Polyalkyl-Polyether-Copolymere bzw. entsprechende Derivate; C_{8/18}-Alkylmono- und -oligoglycoside, ihre Herstellung und ihre Verwendung als oberflächenaktive Stoffe sind beispielsweise aus US 3,839,318, US 3,707,535, US 3,547,828, DE-OS 19 43 689, DE-OS 20 36 472 und DE-A1 30 01 064 sowie EP-A 0 077 167 bekannt. Ihre Herstellung erfolgt insbesondere durch Umsetzung von Glucose oder Oligosacchariden mit primären Alkoholen mit 8 bis 18 C-Atomen.

Als Emulgatoren mit ionischem Charakter kommen anionische, kationische und zwitterionische Emulgatoren in Frage. Anionische Emulgatoren enthalten wasserlöslich machende anionische Gruppen wie z.B. eine Carboxylat-, Sulfat-, Sulfonat- oder Phosphat-Gruppe und einen lipophilen Rest. Hautverträgliche anionische Tenside sind dem Fachmann in großer Zahl bekannt und im Handel erhältlich. Dabei handelt es sich insbesondere um Alkylsulfate oder Alkylphosphate in Form ihrer Alkali-, Ammonium- oder Alkanolammoniumsalze, Alkylethersulfate, Alkylethercarboxylate, Acylsarkosinate sowie Sulfosuccinate und Acylglutamate in Form ihrer Alkali- oder Ammoniumsalze. Auch können Di- und Trialkylphosphate sowie Mono-, Di- und/oder Tri-PEG-alkylphosphate und deren Salze eingesetzt werden.

Auch kationische Emulgatoren können eingesetzt werden. Als solche sind insbesondere quartäre Ammoniumverbindungen verwendbar, etwa Alkltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid. Weiterhin können Monoalklyamidoquats wie z.B. Palmitamidopropyltri-methylammoniumchlorid oder entsprechende Dialkylamidoquats eingesetzt werden. Weiterhin können biologisch gut abbaubare quaternäre Esterverbindungen eingesetzt werden, bei denen es sich meist um quaternierte Fettsäureester auf Basis von Mono-, Di- oder Triethanolamin handelt. Weiterhin können Alkylguanidiniumsalze als kationische Emulgatoren eingesetzt sein.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropylbetain bekannte Fettsäureamid-Derivat. Ebenfalls geeignete Emulgatoren sind ampholytische Tenside.

Unter ampholytischen Tensiden werden solche oberflächenaktiven Verbindungen verstanden, die außer einer C₈/₁₈-Alkyl- oder -Acylgruppe im Molekül mindestens eine freie Aminogruppe und mindestens eine -COOH- oder -SO3H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C 12-18-Acylsarcosin. Neben den ampholytischen kommen auch quatäre Emulgatoren in Betracht, wobei solche vom Typ der Esterquats, vorzugsweise methylquaternierte Difettsäuretriethanolaminester-Salze, besonders bevorzugt sind.

Besonders bevorzugt ist der Einsatz mindestens eines alkoxylierten nichtionischen Emulgators. Dieser nichtionische Basisemulgator oder die Kombination mehrer nichtionischer Emulgatoren kann in einer besonders bevorzugten Ausführung der Erfindung mit ionischen Emulgatorkomponenten kombiniert werden.

Die Mengen an mitverwendeten Ölen und Emulgatoren sind unkritisch für das vorliegende Verfahren und entsprechen den auf den jeweiligen technischen Gebieten verwendeten Formulierungen und sind dem Fachmann bekannt.

Neben den erwähnten Ölen und Emulgatoren können diese Emulsionen dabei dem Fachmann bekannte übliche Hilfs- und Zusatzstoffe enthalten. Dazu gehören beispielsweise Konsistenzgeber, Verdicker, Wachse, UV-Lichtschutzfilter, Antioxidantien, Hydrotrope, Deodorant- und Antitranspirantwirkstoffe, Insektrepellentien, Selbstbräuner, Konservierungsmittel, Parfümöle, Farbstoffe sowie biogene oder synthetische kosmetische Wirkstoffe (wie sie etwa in der Anmeldung DE 10 2005 003 164.1 beschrieben sind).

### Beispiele

### Beispiel 1: Sprühbare kosmetische Lotion

### Schritt 1:

36 g Octylpalmitat (TEGOSOFT^{®} OP, Goldschmidt GmbH), 24 g eines Polyalkoholgemisches mit 12 bis 14 C-Atomen, welches im Mittel acht Ethylenoxideinheiten trägt (C_{12/14}E₈), 10 g Wasser und 30 g Glycerin werden zusammengegeben und verrührt. Es bildet sich eine bei Raumtemperatur einphasige, homogene und transparente Mikroemulsionsphase, deren Einphasengebiet (Winsor-IV-System) im Temperaturbereich zwischen 19 °C und 31 °C liegt.

### Schritt 2:

Die Mikroemulsionsphase wird bei Raumtemperatur in eine fünfmal größere Menge Wasser eingerührt. Es bildet sich eine homogene, milchige, feinteilige O/W-Emulsion. Die so erhaltene Emulsion war im Lagertest bei -15 °C, -5 °C, 5 °C, Raumtemperatur und 40 °C drei Monate stabil.

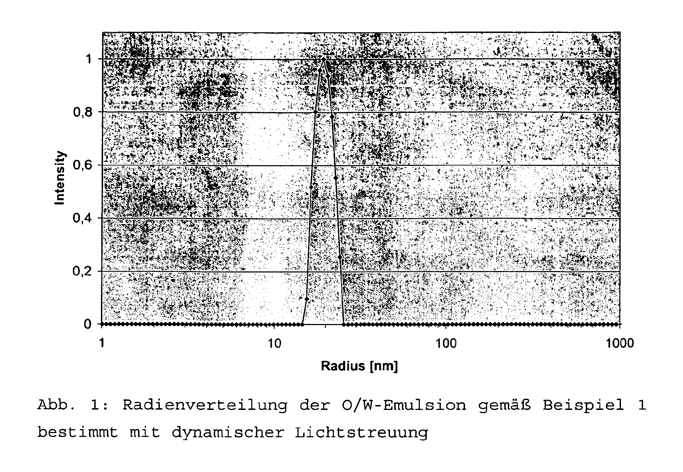

Die Tröpfchengröße der in Schritt 2 erhaltenen O/W-Emulsion wurde nach Verdünnen mit der zwanzigfachen Menge Wasser auf eine Öl/Tensidkonzentration von 0,5 % mit dynamischer Lichtstreuung bestimmt. Abb. 1 zeigt, dass eine enge Verteilung der Tröpfchenradien zwischen 15 nm und 25 nm mit einem Maximum bei 19 nm vorliegt.

### Beispiel 2a: Tränklotion zur Herstellung kosmetischer Feuchttücher

### Schritt 1:

36 g Octylpalmitat (TEGOSOFT^{®} OP, Goldschmidt GmbH), 27 g C_{12/14}E₈, 12 g Wasser, 18 g Glycerin, 3 g Konservierungsmittel (Euxyl^{®} K 300, Schülke & Mair (Phenoxyethanol, Methyl-, Ethyl-, Butyl-, Propyl- und Isobutylparaben)) und 3 g Trilaureth-4-Phospat (Hostaphat^{®} KL 340 D, Clariant) werden zusammengegeben und verrührt. Es bildet sich eine bei Raumtemperatur einphasige, homogene und transparente Mikroemulsionsphase, deren Einphasengebiet (Winsor-IV-System) im Temperaturbereich zwischen 8 °C und 43 °C liegt.

### Schritt 2:

Die Mikroemulsionsphase wird bei Raumtemperatur in eine fünfmal größere Menge Wasser eingerührt. Es bildet sich eine homogene, milchige, feinteilige O/W-Emulsion.

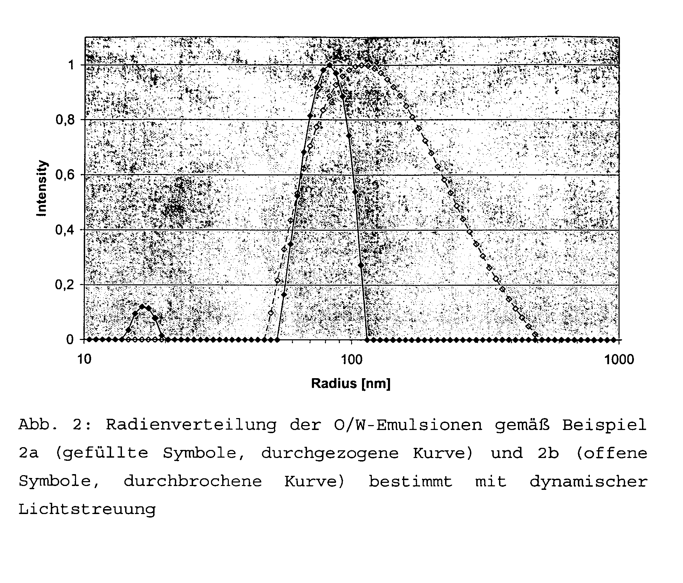

Die Tröpfchengröße der in Schritt 2 erhaltenen O/W-Emulsion wurde nach Verdünnen mit der zwanzigfachen Menge Wasser auf eine Öl/Tensidkonzentration von ca. 0,5 % mit dynamischer Lichtstreuung bestimmt. Abb. 2 zeigt, dass eine enge Verteilung der Tröpfchenradien zwischen 55 nm und 110 nm mit einem Maximum bei 82 nm vorliegt. Überschüssiger Emulgator bildet Micellen, deren Radius zwischen 15 nm und 20 nm liegt.

Zum Vergleich wurde eine O/W-Emulsion gemäß Beispiel 2a ohne Glycerin nach dem PIT-Verfahren hergestellt gemäß

### Beispiel 2b

### Schritt 1:

36 g Octylpalmitat (TEGOSOFT^{®} OP, Goldschmidt GmbH), 27 g C_{12/14}E₈, 12 g Wasser, 3 g Konservierungsmittel (Euxyl^{®} K 300, Schülke & Mair (Phenoxyethanol, Methyl-, Ethyl-, Butyl-, Propyl- und Isobutylparaben)) und 3 g Trilaureth-4-Phospat (Hostaphat^{®} KL 340 D, Clariant) werden zusammengegeben und verrührt. Es bildet sich eine bei Raumtemperatur trübe Emulsion, die sich nach kurzer Zeit in ein zweiphasiges System vom Winsor-I-Typ auftrennt. Bei Erwärmen und Rühren bildet sich ab 70 °C eine einphasige, homogene und transparente Mikroemulsionsphase, deren Einphasengebiet (Winsor-IV-System) im Temperaturbereich zwischen 70 °C und 85 °C liegt.

### Schritt 2 (PIT-Verfahren) :

Die Mikroemulsionsphase wird in einem Wasserbad bei Raumtemperatur abgeschreckt. Es bildet sich eine homogene, transparente, feinteilige O/W-Emulsion.

Die gemäß Schritt 2 erhaltene O/W-Emulsion wurde gemäß Beispiel 2a auf eine Öl/Tensidkonzentration von ca. 0,5 % verdünnt und die Tröpfchengröße mit dynamischer Lichtstreuung bestimmt. Abb. 2 zeigt, dass eine breite Verteilung der Tröpfchenradien zwischen 50 nm und 490 nm mit einem Maximum bei 110 nm vorliegt.

### Mikroskopie:

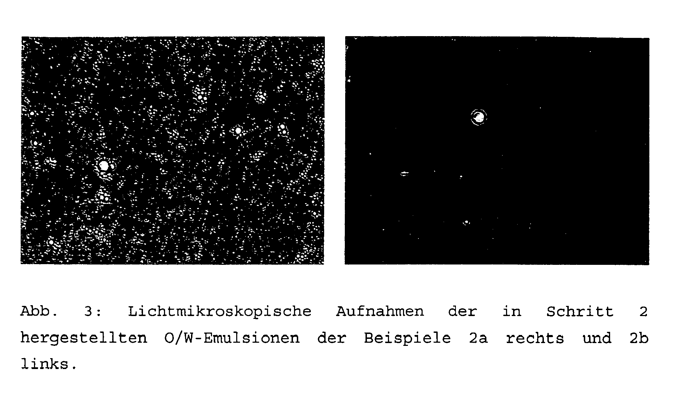

Die in Schritt 2 der Beispiele 1 und 2 hergestellten feinteiligen O/W-Emulsionen wurden unter dem Lichtmikroskop bei vierzigfacher Vergrößerung betrachtet. Abbildung 3 zeigt, dass die nach dem PIT-Verfahren gemäß Beispiel 2b hergestellte Emulsion neben Luftbläschen Tröpfchen im Submikrometerbereich enthält, während sich bei der nach dem PSQ-Verfahren gemäß Beispiel 2a hergestellten Emulsion ein homogenes Bild ergibt, weil die Tröpfchengöße unterhalb der Auflösung des Mikroskops liegt.

## Patentansprüche

1. Verfahren zur Herstellung von feinteiligen Öl-in-Wasser-Emulsionen, die Öl, Wasser und mindestens einen Emulgator enthalten, **dadurch gekennzeichnet, dass** es einen Schritt
A) Herstellen einer Mischung 2, die Öl, Wasser, mindestens einen Emulgator und mindestens eine kosmotrope Substanz aufweist, durch Mischen von Öl, Wasser, mindestens einem Emulgator und mindestens einer kosmotropen Substanz, wobei die Phaseninversionstemperatur PIT2 dieser Mischung (Winsor-IV-System) kleiner ist als die Phaseninversionstemperatur PIT1 einer Mischung 1 (Winsor-IV-System), die keine kosmotropen Substanzen und ansonsten die gleiche Zusammensetzung wie Mischung 2 aufweist,
und nachfolgend einen Schritt
B) Zugabe eines Verdünnungsmittels zur Mischung 2 zur Überführung dieser Mischung in eine Emulsion 3, wobei die Menge des zugegebenen Verdünnungsmittels so gewählt ist, dass die erhaltene Emulsion 3 bei einer vorgegebenen Temperatur nicht im Winsor-IV-Phasengebiet liegt,
umfasst.

2. Verfahren nach Anspruch 1, **dadurch gekennzeichnet, dass** als Emulsion 3 eine bei Umgebungs-, Verarbeitungs- oder Verwendungstemperatur kinetisch stabile Emulsion hergestellt wird.

3. Verfahren nach Anspruch 1 oder 2, **dadurch gekennzeichnet, dass** die feinteilige Emulsion 3 eine mittlere Tröpfchengröße von kleiner 1 µm aufweist.

4. Verfahren nach zumindest einem der Ansprüche 1 bis 3, **dadurch gekennzeichnet, dass** als Mischung 2 eine Mikroemulsion hergestellt wird.

5. Verfahren nach zumindest einem der Ansprüche 1 bis 4, **dadurch gekennzeichnet, dass** in Schritt B) soviel Verdünnungsmittel zugegeben wird, dass die Übergangstemperatur, bei der die Emulsion 3 in das Winsor-IV-Phasengebiet übergeht, zumindest 1 K oberhalb der Umgebungs-, der Verarbeitungs- oder Verwendungstemperatur liegt.

6. Verfahren nach zumindest einem der Ansprüche 1 bis 5, **dadurch gekennzeichnet, dass** in Schritt B) zu 1 Massenteil der Mischung 2 mindestens 1 Massenteil Verdünnungsmittel zugegeben wird.

7. Verfahren nach zumindest einem der Ansprüche 1 bis 6, **dadurch gekennzeichnet, dass** als Verdünnungsmittel in Schritt B) Wasser oder eine wässrige Lösung eingesetzt wird.

8. Verfahren nach zumindest einem der Ansprüche 1 bis 7, **dadurch gekennzeichnet, dass** als Emulsion 3 eine bei Umgebungs-, Verarbeitungs- oder Verwendungstemperatur kinetisch stabile Emulsion hergestellt wird, wobei in Schritt A) eine thermodynamisch stabile und makroskopisch homogene Mischung aus Wasser, Öl, mindestens einem Emulgator und mindestens einer kosmotropen Substanz nach üblichen Verfahren hergestellt wird, und wobei der Schritt A) bei einer Temperatur durchgeführt wird, die kleiner als die Phaseninversionstemperatur PIT1 der Mischung 1 ohne Zugabe der kosmotropen Substanzen ist.

9. Verfahren nach zumindest einem der Ansprüche 1 bis 7, **dadurch gekennzeichnet, dass** als Emulsion 3 eine bei Umgebungs-, Verarbeitungs- oder Verwendungstemperatur kinetisch stabile Emulsion hergestellt wird, wobei Schritt A) das Zumischen mindestens soviel zumindest einer kosmotropen Substanz zu einer Mischung 1, die Öl, Wasser und mindestens einen Emulgator enthält und die eine Phaseninversionstemperatur PIT1 aufweist (Winsor-IV-System), so dass eine Mischung 2 erhalten wird, deren Phaseninversionstemperatur (Winsor-IV-System) PIT2 kleiner ist als PIT1, umfasst.

10. Verfahren nach zumindest einem der Ansprüche 1 bis 7, **dadurch gekennzeichnet, dass** als Emulsion 3 eine bei Umgebungs-, Verarbeitungs- oder Verwendungstemperatur kinetisch stabile Emulsion hergestellt wird, wobei in Schritt A) aus Wasser, Öl, mindestens einem Emulgator und mindestens einer kosmotropen Substanz eine W/O-Mikroemulsionsphase im Gleichgewicht mit überschüssiger Wasserphase (Typ W II) nach üblichen Verfahren ohne Überschreitung der ursprünglichen Phaseninversionstemperatur hergestellt wird.

11. Verwendung eines Verfahrens gemäß mindestens einem der Ansprüche 1 bis 10 zur Herstellung von Emulsionen bei der Herstellung von kosmetischen, dermatologischen, pharmazeutischen oder agrochemischen Zubereitungen.

12. Verwendung eines Verfahrens gemäß mindestens einem der Ansprüche 1 bis 10 zur Herstellung von Emulsionen bei der Herstellung von Tränktüchern oder in sprühbaren Zubereitungen für Gesichts- und Körperpflege, Babypflege, Sonnenschutz, Make up-Remover, Antiperspiratien/Deodorantien.

13. Verwendung eines Verfahrens gemäß mindestens einem der Ansprüche 1 bis 10 zur Herstellung von Emulsionen bei der Herstellung wässriger Formulierungen für Applikationen in den Bereichen Haushalt, Sport, Freizeit und Industrie.

14. Verwendung eines Verfahrens gemäß mindestens einem der Ansprüche 1 bis 10 zur Herstellung von Emulsionen bei der Herstellung von Tränktüchern oder in sprühbaren Zubereitungen zur Reinigung und Pflege von Textilien, Leder, Kunststoffen, metallischen und nichtmetallischen Oberflächen.

15. Verwendung eines Verfahrens gemäß mindestens einem der Ansprüche 11 bis 10 zur Herstellung von Emulsionen bei der Herstellung von sprühbaren Zubereitungen von agrochemischen Formulierungen, die Öle und gegebenenfalls weitere Aktivstoffe enthalten.

16. Feinteilige Öl-in-Wasser-Emulsion, erhältlich durch ein Verfahren gemäß zumindest einem der Ansprüche 1 bis 10.

17. Emulsion gemäß Anspruch 16 **dadurch gekennzeichnet, dass** sie eine Teilchengröße von kleiner 1 µm aufweist.
